Europäisches Patentamt

European Patent Office

Office européen des brevets

(19)

(11) Numéro de publication: **0 099 775**
**B1**

(12) # FASCICULE DE BREVET EUROPÉEN

(45) Date de publication du fascicule du brevet:
15.10.86

(51) Int. Cl.⁴: **A 61 K 31/165**

(21) Numéro de dépôt: **83401236.1**

(22) Date de dépôt: **16.06.83**

(54) Application du N-(diéthylaminoéthyl) 2-méthoxy-5-méthylsulfonyl benzamide dans l'inhibition de la tryptophane pyrrolase.

(30) Priorité: **17.06.82 FR 8210565**

(43) Date de publication de la demande:
**01.02.84 Bulletin 84/5**

(45) Mention de la délivrance du brevet:
**15.10.86 Bulletin 86/42**

(84) Etats contractants désignés:
**AT BE CH DE FR GB IT LI LU NL SE**

(56) Documents cité:
**FR-A-2 305 176**

(73) Titulaire: **SOCIETE D'ETUDES SCIENTIFIQUES ET INDUSTRIELLES DE L'ILE DE FRANCE, 46, Boulevard de Latour- Maubourg, F-75340 Paris Cedex 07 (FR)**

(72) Inventeur: **Besançon, Denis, Dr., 31, Boulevard de Latour- Maubourg, F-75007 Paris (FR)**

(74) Mandataire: **Gallochat, Alain, SOCIETE D'ETUDES SCIENTIFIQUES ET INDUSTRIELLES DE L'ILE DE FRANCE 46, Boulevard de Latour Maubourg, F-75340 Paris Cedex 07 (FR)**

**Description**

La présente invention concerne l'utilisation du N-diéthylaminoéthyl) 2-méthoxy 5-méthylsulfonyl benzamide (D.C.I. = tiapride) pour la fabrication d'un médicament servant à l'inhibition de la tryptophane pyrrolase et au traitement du syndrome dépressif du sevrage alcoolique.

Des études pharmacologiques ont montré que l'affinité du Tiapride sur les récepteurs dopaminergiques était augmentée lorsque ces récepteurs étaient sensibilisés (Fortune et al. 1980). Des essais in vivo, utilisant comme modèle le comportement de rotation provoqué chez la souris par des lésions 6-hydroxydopamine (6-OHDA) ont montré que le Tiapride antagonisait très fortement ce comportement.

Comme la 6-OHDA augmente la sensibilité des récepteurs, ce résultat a suggéré un effet plus marqué du Tiapride dans des conditions d'hypersensibilité. Il a également été montré qu'une administration chronique d'éthanol provoquait une hypersensibilité des récepteurs dopaminergiques. Les études menées par Engel et al., Liljequist et al., qui ont soumis de jeunes rats au sevrage à une intoxication chronique par l'éthanol administration à des concentrations croissantes (16-24 %) pendant 270 jours, ont montré une augmentation sensible de l'activité motrice après une application bilatérale de dopamine dans le nucleus accumbens.

Ces données ont justifié la recherche de possibles effets thérapeutiques du Tiapride sur divers désordres comportementaux tels que l'agitation, l'agressivité, ainsi que les tremblements, observés chez l'homme dans l'alcoolisme.

Les observations cliniques qui ont suivi ont confirmé l'activité du Tiapride dans ce domaine, des effets favorables ayant été également observés dans l'insomnie, l'anxiété et l'irritabilité, de même qu'un ralentissement des nausées et un regain d'appétit; les douleurs dues aux polynévrites alcooliques se sont également calmées. L'effet global résulte en une acceptation plus grande de la part des patients pour traiter leur alcoolisme.

Le Tiapride a donc été utilisé chez l'alcoolique pour le traitement des troubles du comportement (irritabilité, agitation), des tremblements, des troubles gastro-intestinaux, toute cette symptomatologie pouvant être reliée à une hypersensibilité des récepteurs dopaminergiques.

Lors de recherches complémentaires menées à l'initiative de la demanderesse, il s'est révélé que le Tiapride, dans certaines conditions, pouvant inhiber l'activité d'une enzyme hépatique, la tryptophane pyrrolase.

Il convient de noter que l'inhibition d'une enzyme n'a rien de commun avec un antagonisme au niveau du récepteur d'un neuromédiateur tel que la dopamine; de plus il n'est pas possible d'envisager quel serait le mécanisme de cette inhibition enzymatique en partant des résultats et/ou des propriétés de l'antagonisme précité.

Les tableaux et schémas suivants illustrent bien l'action du Tiapride comme inhibiteur de la tryptophane pyrrolase.

**Evolution dans le temps des effets du Tiapride sur l'activité tryptophane pyrrolase dans le foie du rat**

Activité tryptophane pyrrolase ($\mu$ mole de kynurénine formée/h et par g de foie frais)

| Temps (h) | Activité Holoenzyme | Activité enzyme totale | Activité Apoenzyme |
|---|---|---|---|
| 0 | $2,7 \pm 0,26$ | $6,3 \pm 0,05$ | $3,6 \pm 0,21$ |
| 0,5 | $1,7 \pm 0,10$[*] | $3,7 \pm 0,27$[***] | $2,0 \pm 0,16$[***] |
| 1 | $1,2 \pm 0,06$[**] | $2,9 \pm 0,22$[***] | $1,7 \pm 0,17$[***] |
| 2 | $0,9 \pm 0,00$[***] | $2,3 \pm 0,06$[***] | $1,4 \pm 0,06$[***] |
| 3 | $1,5 \pm 0,05$[**] | $3,8 \pm 0,47$[**] | $2,3 \pm 0,29$[*] |
| 4 | $1,8 \pm 0,11$[*] | $4,6 \pm 0,14$[***] | $2,8 \pm 0,09$[*] |
| 5 | $2,4 \pm 0,11$ | $6,1 \pm 0,64$ | $3,7 \pm 0,53$ |

Dose de Tiapride: 10 mg/kg, I.P. dans 2 ml d'eau salée.
Rats mâles Wistar - Nombre de rats par groupe: 4
Signification des différences: [*]$P < 0,82$ [**]$P < 8,005$ [***] $P < 0,001$
Ces résultats sont rassemblés sur les courbes suivantes
Activité tryptophane
pyrrolase

2

Activité tryptophane pyrrolase

Temps (h)

x = Holoenzyme
o = Total
• = Apoenzyme

**Effets de diverses doses dé Tiapride sur l'activite tryptophane pyrrolase dans le foie du rat**

Activité tryptophane pyrrolase ($\mu$ mole de kynurénine formée/h et par g de foie frais)

| Temps (h) | Activité Holoenzyme | Activité enzyme totale | Activité Apoenzyme |
|---|---|---|---|
| 0 | $2,7 \pm 0,09$ | $6,3 \pm 0,33$ | $3,6 \pm 0,25$ |
| 0,5 | $1,9 \pm 0,07$[***] | $4,3 \pm 0,15$[**] | $2,4 \pm 0,09$[**] |
| 1 | $1,7 \pm 0,02$[***] | $3,0 \pm 0,06$[***] | $1,3 \pm 0,07$[***] |
| 2,5 | $1,2 \pm 0,08$[***] | $2,5 \pm 0,11$[***] | $1,3 \pm 0,06$[***] |
| 5 | $1,2 \pm 0,11$[***] | $2,5 \pm 0,11$[***] | $1,3 \pm 0,06$[***] |
| 7,5 | $1,2 \pm 0,09$[***] | $2,5 \pm 0,14$[***] | $1,3 \pm 0,07$[***] |
| 10 | $1,1 \pm 0,06$[***] | $2,4 \pm 0,22$[***] | $1,3 \pm 0,19$[***] |

Intervalle de temps après l'injection I.P.: 2 h.
Chaque groupe comprenait 4 rats mâles de souche Wistar.
Signification des différences: [**] P < 0,885 [***] P < 0,001
Activité tryptophane
pyrrolase

**Activité tryptophane pyrrolase**

• = Holoenzyme
o = Total
x = Apoenzyme

**Doses de Tiapride (mg/kg)**

Doses de Tiapride (mg/kg)

Il ressort de ce qui précède que le Tiapride conduit bien à une inhibition de l'activité tryptophane pyrrolase, avec une dose efficaoe de 2,5 mg/kg, ce qui correspond chez l'adulte à une dose de 150-175 mg.

La demanderesse a alors étudié dans quelle mesure le Tiapride pouvait avoir une application thérapeutique decoulant de l'inhibition de l'enzyme hépatique.

L'étude de la littérature montre que l'alcoolique présente un état dépressif lors du sevrage. Or il est connu que les modifications d'humeur chez l'homme sont en rapport notamment avec des mécanismes cérébraux impliquant la sérotonine comme neuromédiateur. La disponibilité en cette monoamine dépend du taux du précurseur plasmatique de la sérotonine, le tryptophane libre.

L'alcoolisme chronique inhibe l'activité tryptophane pyrrolase par augmentation du NRD(P)H alors que le sevrage alcoolique augmente l'activité tryptophane pyrrolase par un mécanisme hormonal impliquant la libération de corticostérone; il s'ensuit qu'au cours du sevrage alcoolique, il se produit une baisse importante de la disponibilité en sérotonine qui est responsable du syndrome dépressif.

La demanderesse a donc recherché si le Tiapride, en inhibant l'activité tryptophane pyrrolase, pouvait avoir une efficacité dans le syndrome dépressif du sevrage alcoolique.

Cette étude a été réalisée en Grande-Bretagne par D.J. MURPHY et G.K. SHAW, sous forme d'un essai double insu comparant les effets du Tiapride et du Clométiazole, substance communément utilisée dans le sevrage alcoolique, ces composés étant administrés aux doses suivantes:

|  | | Quantités maximales autorisées par jour | | Quantités totales utilisées en moyenne chaque jour | |
|---|---|---|---|---|---|
|  | | Tiapride | Clométiazole | Tiapride | Clométiazole |
| 1° jour | | 800 mg | 3,072 g | 400 mg | 1,824 g |
| 2° jour | | 800 mg | 3,072 g | 700 mg | 2,592 g |
| 3° jour | | 600 mg | 2,304 g | 500 mg | 2,112 g |
| 4° jour | | 450 mg | 1,728 g | 400 mg | 1,536 g |
| 5° jour | | 300 mg | 1,152 g | 250 mg | 0,96 g |

4

Les patients ont été inclus dans l'un ou l'autre groupe de traitememt par randomisation après séparation suivant l'âge, le sexe et la concentration d'alcool dans le sang au moment de l'admission à l'hôpital; la durée de l'étude a été de cinq jours.

L'état dépressif a été jugé tous les jours en utilisant la méthode d'évaluation de WAKEFIELD (SRAITH et al. 1971), version modifiée et abrégée de l'échelle d'autoévaluation de la dépression de ZUNG (ZUNG, 1965). L'évaluation de WAKEFIELD comporte douze items cotés de 0 à 3 ce qui aboutit à un score total compris entre 0 et 36; cette méthode d'évaluation a une bonne fiabilité lorsqu'elle est répétée.

En raison de leur intoxication tous les patients ne furent pas capables de remplir leur échelle d'évaluation du 1er jour; sur 16 patients traités par le Tiapride, le score moyen du 1er jour a été 23 (fourchette 0 à 35), sur 15 traités par le Clométiazole, le score moyen a été 24 (fourchette 3 à 36). Au 5ème jour les scores respectifs ont été de 14 (fourchette 0 à 30) et 19 (fourchette de 0 à 31).

45 patients ont été évalués au 5ème jour; la différence sur les scores finaux obtenus n'est pas statistiquement significative selon le v test de MANN WHITNEY. Cependant comme l'évaluation de WAKEFIELD est prévue pour mesurer la dépression endogène et n'est pas parfaitement adaptée aux évaluations journalières, les réponses à l'item 1 de l'évaluation de WAKEFIELD ont été analysées comme un témoin de l'humeur dépressive. Dans cet item 1 l'assertion "je me sens misérable et triste" appelle 4 réponses: oui absolument, oui parfois, non pas tellement, non pas du tout, que le patient choisit. La proportion de patient dans les 2 groupes répondant oui est montrée dans la figure suivant:

% de patiens répondant OUI à l'item 1

% de patients répondant OUI à l'item 1

La proportion des cas dans lesquels les patients ont répondu OUI par rapport aux cas où ils ont répondu NON est, dans le groupe Tiapride, de 48-56; cette même proportion est de 68-36 dans le groupe Clométiazole, d'où une différence hautement significative puisque l'on obtient:

chi carré = 6,582, p < 0,02

En conclusion, il apparait donc que le Tiapride est efficace dans le syndrome dépressif du sevrage alcoolique.

**Revendications**

1) Utilisation du N-(diéthylaninoéthyl) 2-méthoxy 5-méthylsulfonyl benzamide pour la fabrication d'un médicament servant à l'inhibition de la tryptophane pyrrolase.

2) Utilisation du N-(diéthylaninoéthyl) 2-méthoxy 5-méthylsulfonyl benzamide pour la fabrication d'un médicament servant dans le traitement du syndrome dépressif du sevrage alcoolique.

**Patentansprüche**

1. Verwendung des N-(Diethylaminoethyl)-2-methoxy-5-methyl-sulfonylbenzamids für die Herstellung eines zur Inhibierung der Tryptophanpyrrolase dienenden Arzneimittels.

2. Verwendung des N-(Diethylaminoethyl)-2-methoxy-5-methyl-sulfonylbenzamids für die Herstellung eines zur Behandlung des Depressionssyndroms beim Alkoholentzug dienenden Arzneimittels.